Europäisches Patentamt

European Patent Office

Office européen des brevets

(11) Publication number: **0 385 373**
**A2**

## EUROPEAN PATENT APPLICATION

(21) Application number: **90103749.9**

(22) Date of filing: **26.02.90**

(51) Int. Cl.5: **A61K 35/12**

(30) Priority: **27.02.89 IL 89431**

(43) Date of publication of application:
**05.09.90 Bulletin 90/36**

(84) Designated Contracting States:
**CH DE ES FR GB IT LI NL**

(71) Applicant: **YEDA RESEARCH AND DEVELOPMENT COMPANY LIMITED**
**P.O. Box 95**
**Rehovot 76100(IL)**

(72) Inventor: **Ben-Nun, Avraham**
**13 Hashita Street**
**Yavne(IL)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Treatment of autoimmune diseases.**

(57) The invention relates to a pharmaceutical composition for the treatment of, and for the prevention of autoimmune disease in humans and animals. The composition comprises as active ingredient an effective quantity of cells expressing MHC molecules of the patients, or other cells compatible with such cells, prepared by incubating them with tissue homogenate, or with total or partial tissue extract from the afflicted organ of another mammal, or with purified antigens or fragments thereof specific for such autoimmune disease so as to present at their surface antigens for such autoimmune disease. Optionally cells which have been subjected to oxidation or cross-linking or both and membranes or membrane fragments thereof, can be used as highly effective ingredient for the treatment of the disease. Cells used are B lymphocytes, macrophages, endothelial cells, dendritic cells astrocytes and the like expressing MHC molecules or adapted to express MHC molecules and to present antigens for T lymphocytes.

The invention furthermore relates to a process for the production of the active ingredients of such compositions.

EP 0 385 373 A2

## Field of the invention:

The present invention relates to a pharmaceutical composition for the treatment of, and for the prevention of, autoimmune disease in humans and mammals, based on the efficiency of such a composition in the treatment of experimental autoimmune diseases. Autoimmune diseases are caused by abnormal attack of the immune system against an individual's own tissue. Unlike the case of experimental autoimmune diseases in laboratory animals, the specific target antigen in the human afflicted target tissue is largely unknown. As a result, the pathogenic lymphocytes or the antigenic epitope recognized by these destructive lymphocytes has not yet been defined for most human autoimmune disease. In the absence of such critical information, it is most difficult to design an immunespecific therapy. The pharmaceutical composition herein, comprises as active ingredient an effective quantity of cells of patient, expressing major histocompatibility (MHC) molecules, or of other cells compatible with such cells, incubated with a tissue extract from afflicted organ of patient, or from an equivalent organ of another mammal. Such a composition is effective in the treatment of autoimmune disease regardless of how much we know about the etiology of the disease, the specific target antigen in the afflicted organ or the immune pathogenic cells. The cells can be subjected to a further step of oxidative treatment and/or to a step of cross-linking or fixation. Instead of cells, only the cell membranes can be used.

## Background of the invention

Autoimmiune diseases share the common feature of being caused by the attack of the immune system against individual's own tissue(s), normally being recognized as self and against which the immune system is normally tolerant.

The etiological agents of autoimmune diseases are endogenous T or B lymphocytes which attack normal constituents of the individual. Among such autoimmune diseases there may be mentioned systemic lupus erythematosus and myesthenia gravis as examples for diseases believed to be attributed to pathogenic B lymphocytes, and multiple sclerosis, rheumatoid arthritis, some forms of thyrioditis and diabetes mellitus as an example of autoimmune diseases believed to be attributed to pathogenic T lymphocytes.

Experimental autoimmune encephalomyelitis (EAE) is a neurological autoimmune disease that can be induced in genetically susceptible animals by the injection of brain or spinal cord homogenate, or purified basic protein of the myelin (MBP), or by an encephalitogenic peptide derived from MBP, when emulsified in an appropriate adjuvant. In species such as guinea pigs, rats and mice, EAE is usually manifested as an acute, monophasic disease characterized by hind leg paralysis, accompanied by considerable infiltration of leukocytes into the central nervous system (CNS), from which most of the animals die or spontaneously recover. However, under certain circumstances, chronic progressive or chronic relapsing forms of EAE can be induced, with demyelination reminiscent of mutiple sclerosis (MS) in humans. EAE has been a subject of intensive studies, both as a model for human demyelinating autoimmune diseases such as MS and as prototypic organ-specific $CD_4^+$-T-cell mediated autoimmune diseases.

In MS as well as for most other human organ-specific T cell mediated autoimmune diseases, the etiology of the disease has not been identified. Nevertheless, the common features of the clinical and pathological manifestations of the chronic relapsing form of EAE and MS in humans have made EAE an acceptable experimental model for MS.

EAE is a $CD_4^+$-T-cell mediated autoimmune disease. $CD_4^+$-T-cells, via their $\alpha\beta$ receptor, recognize ligands composed of a fragment of a foreign antigen or an autoantigen complexed to a product of the major histocompatibility complex (MHC), expressed on antigen-presenting cells. This phenomenon is known as an MHC restriction. Polymorphic amino acid residues within the MHC molecule control both the binding of antigen fragments and the interaction of the Ag/MHC complex with the $\alpha\beta$ T cell receptor indicating that the receptor contact both the antigen and MHC molecule composing the receptor ligand. Recent reports of the three-dimensional structure of the class I human MHC molecule has provided a potential explanation for this tri-complex interaction-the Ag/MHC and T- cell receptor.

Our increasing knowledge about the molecular basis of T cell function should pave the way for the design of specific competitive synthetic peptides or monoclonal antibodies capable of ameliorating T-cell mediated pathogenesis. Nevertheless, there is as yet no immune-specific cure for autoimmune diseases.

Based on studying EAE mainly in mice and rats, several approaches to therapy of autoimmune diseases have been proposed. These attempts at therapy in EAE have involved treatment with cyclophosphamide (1), injection of monoclonal antibodies against T cell surface markers (2), of antibodies against the class II MHC molecule (3), and by antibodies against activation antigens.

More immune-specific attempts at therapy of EAE, as an experimental model system for organ-specific autoimmune diseases mediated by T lymphocytes have included systemic injections of soluble MBP (4) or of a synthetic copolymer (COP 1) somehow crossreactive with MBP (5).

As more sophisticated highly immune specific attempts at therapy in EAE include a) the possibility, to isolate the pathogenic T lymphocytes specific to MBP and to use them in an attenuated form to vaccinate against EAE (6); b) the recently demonstrated restricted usage of $V\beta$ elements by the MBP specific T cells allowing the use of monoclonal antibodies anti $V\beta$ elements to block the disease (7,8); c) the recently demonstrated possibility to vaccinate against EAE by peptides derived from the $V\beta$ gene products used by MBP-specific T cells (9,10); and d) the possibility to design synthetic peptides competitive with the encephalitogenic epitope of MBP for binding to the MHC molecule or to TcR (11,12).

The major difficulty of such technologically sophisticated approaches is that these techniques require a great deal of knowledge and information which is presently largely unknown for most human autoimmune diseases: Unlike experimental autoimmune diseases such as EAE, experimental autoimmune thyroiditis (EAT) and collagen (type II) induced arthritis (CIA) or others, the specific target antigen is largely unknown for most human autoimmune diseases. As a result, only a very little information is available for the design of specific competitive synthetic peptides, or monoclonal antibodies capable of ameliorating T-cell mediated pathogenesis. Such information concerns the specific target antigen in the afflicted organ, the specific epitope on the target antigen and the specific MHC allele recognized as a complex by the pathogenic T lymphocytes, and the $V\alpha$ or $V\beta$ elements of the TcR involved in the immune recognition by pathogenic T lymphocytes. Furthermore, due to extreme polymorphism between individuals each of these factors, even if known, may vary from one individual to another bearing the same autoimmune disease. There is provided here a means for therapy of autoimmune diseases which overcomes these limitations, and which is effective in the treatment of EAE, EAT and CIA in rodents as experimental model systems for human autoimmune diseaes.

Summary of the invention

According to the present invention there is provided a pharmaceutical composition, methods of preparation and methods of administration, for the prevention and treatment of autoimmune diseases in humans and mammals, regardless of the etiology of the autoimmune disease or the antigenic moiety in an afflicted organ towards which immune system is operating abnormally in a destructive manner. A pharmaceutical composition for the treatment of, and for the prevention of, autoimmune disease in humans, which comprises as active ingredient an effective quantity of MHC molecules expressing cells of the patient, or of membranes or membrane fragments of such cells, or of cells which are compatible with such cells, which cells or membranes present at their surface, antigens specific for such autoimmune disease, said antigens being derived from tissue homogenate, a total or partial tissue extract, or purified protein, or antigen, or fragments thereof, specific for such atuoimmune disease,which cells or membranes or membrane fragments have optionally been subjected to oxidation or cross-linking or both. Preferably the cells used are B lymphocytes, macrophages (Mφ), endothelial cells, astrocytes or any other such type of cells adapted to present at their surface antigens for T lymphocytes involved in the autoimmune disease.

The compositions are specially intended for the treatment of an autoimmune disease which is organ-specific and where the specific antigen from such organ may, or may not, be known. According to a preferred embodiment the cells used are subjected to an oxidative treatment and/or treatment with cross-linking or fixing agents and instead of whole cells, membranes of such cells can be used. The invention further relates to a process for the production of MHC molecules expressing cells or membranes, capable of presenting antigens to lymphocytes involved in the disease, for use as active ingredient in a composition, which comprises incubating a tissue homogenate or extract or a protein fraction, total or partial, from the afflicted organ of a patient, or preferably from an equivalent organ of another mammal or a purified protein or antigen or a fragment thereof, with said cells or cells adapted to present at their surface antigens for T lymphocytes which cells or membranes or membrane fractions have optionally been subjected to oxidation or cross-linking or both. Advantageously, the organ extract is produced by decomposing the organ tissue to be used as a homogenate or as a total or partial tissue extract. In cases of autoimmune diseases where the specific autoimmune antigen is well defined or where the pathogenic epitope is known, the said antigen or fraction of the antigen comprising the pathogenic epitope of the antigen can be incubated with the cells to produce whole ingredient effective in treatment of, and prevention of, autoimmune disease. The cells can be subjected to a further step of oxidative treatment and/or the additional step of cross-slinking or fixation. Instead of whole cells, only the membrane or membrane fractions of such cells can be used as active ingredient effective in the treatment and prevention of autoimmune disase. The compositions are in injectable forms and are effective when injected intraperitoneally, intravenously or subcutaneously, or when administered orally or as an aerosol.

Active ingredients prepared for the treatment of, and for the prevention of, autoimmune diseases, as described here and administered as described here, were effective in the blocking and treatment of experimental autoimmune encephalomyelitis (EAE), experimental autoimmune thyroiditis (EAT) and collagen (type II) induced arthritis (CIA) in rodents as an experimental model systems for organ-specific autoimmune diseases in humans.

## Description of the preferred embodiment

The invention relates to the preparation of pharmaceutical compositions for the treatment of any organ specific autoimmune disease in humans and mammals, regardless of the etiology of the autoimmune disease or the antigenic moiety in the afflicted organ towards which the immune system is operating abnormally in a destructive manner, and regardless of the type of pathogenic lymphocytes involved in the pathogenic process.

This invention is based on the successful treatment of experimental autoimmune encephalomyelitis (EAE) in SJL/J mice, as an experimental model system, by macrophages, or B cells, expressing MHC molecules and especially class II MHC molecules on their surface, that were pulsed with brain homogenate or purified basic protein of the myelin (MBP), or with synthetic peptide encompassing the encephalitogenic epitope. These cells can be macrophages, B cells, dendritic cells, endothelial cells, astrocytes epithelial cells or any other cell type adapted to express the MCH molecules and to be functional in what is known as MHC-restricted antigen presentation to T lymphocytes. Although exemplified with reference to class II MHC molecules, it ought to be understood that the invention applies also to other MHC molecules.

## Preparation of organ tissues homogenate

For the treatment of EAE in mice, the mouse spinal cord homogenate was prepared as follows so as to be accessible for the cells: The mouse spinal cord was homogenized in phosphate buffered saline (PBS) in a weight to volume ratio of 1:1, using a homogenizator or blender. The homogenate was then sonicated to break the tissue into smaller fragments and sieved through a very fine nylon mesh to remove large pieces. the homogenate was then lyophilized and kept at -20°. Another procedure which was cleaner and also effective in obtaining a fairly good representation of brain antigens was to prepare a protein extract from the brain tissue in the usual chloroform:methanol defatting methods, and the protein extract was lyophilized. Before use for pulsing the macrophages or B cells or the like, a portion of the extracts was resuspended in PBS.

For treatment of patients, the source for the afflicted organ can be any mammal close to a human being, as most of the antigens involved in organ specific autoimmune disease are fairly conserved among species. Thus, monkey, porcine, bovine, sheep etc. organs may be used for preparation as homogenate or protein extract. For the treatment of EAE in rats, myelin basic protein of rats, mice or guinea pigs, or the peptide constituting the 72-84 aa residues of guinea pig myelin basic protein are also effective. In mice, myelin basic proteins of rats, mice, guinea pigs and bovine, or the peptide constituting 89-101 aa residues of MBP are effective in the treatment of EAE.

## Preparation of the cells

Macrophages or B-cells or other cell types as mentioned above used for the treatment have to be autologous or syngeneic with the patient in their MHC. The cells, if not constitutively expressing high levels of MHC molecules, can be adapted or induced to do so by incubating them with gamma-interferon (100 µ/ml).

For the treatment of EAE in SJL/J mice there were used peritoneal macrophages obtained following injections of thioglycolate by conventional methods or LPS-stimulated B-cells obtained from the spleen, or astrocytes that were derived from SJL/J mice. Only the macrophages and astrocytes required a treatment with gamma-interferon for expressing higher levels of MHC molecules. The cells, in order to be effective in the treatment of EAE were washed in PBS and incubated (1 -10 x $10^6$/ml) for 1 - 24 h with a preparation of MSCH homogenate (0.01 - 1 mg/ml or with MSCH protein extract (0.001 - 0.5 mg/ml) or with purified MBP (0.001 -0.1 mg/ml) or with synthetic peptide constituting 89-101 aa residues of MBP (0.01 - 500 ug/ml) in RPMI medium and 0.5% fresh autologous serum and antibiotics L-glutamine and 2 mercaptoethanol as usual. The cells were then washed in PBS to remove excess of homogenate or proteins.

Membrane preparation

Macrophages prepared as mentioned above were cultured to confluency for 16 h, in 100 mm plastic petri dish in RPMI medium as above, containing gamma interferon (200 IU/ml) and MBP, or MSCH or peptide as above. The macrophages were washed free of excess of antigens and membranes were prepared by hypotonic treatment as described (13) and membranes were isolated by sucrose-gradient centrifugation (13). Membranes were prepared from astrocytes in a similar manner(13). LPS-stimulated B cells that were pulsed with antigen or tissue extract as above were also prepared by hypotonic shock and sucrose-gradient isolation.

As elaborated in the Examples, these cells that had been pulsed with the organ homogenate or protein extract or purified MBP or with peptide fragment can be effective as such, or after modification by oxidative agents, or by cross-linking or fixative agent, or by cross-linking and oxidative treatment. Membranes and membrane fragments derived from said treated cells were also found to be effective for the treatment of EAE.

As further elaborated in the Examples, such an appropriate composition can be effective also in the treatment of other experimental autoimmune diseases, such as experimental autoimmune thyroiditis (EAT) and collagen (type II) induced arthritis (CIA).

Experimental models

EAE can be induced in genetically susceptile strains of mice or rats by immunizing them with whole brain tissue or by the purified basic protein of myelin (MBP) or by encephalitogenic peptide emulsified in complete Freund's adjuvant (CFA). EAE is usually manifested as an acute disease charaterized by paralysis and cellular infiltration in the white matter of the central nervous system. Under certain circumstances, a chronic relapsing form of EAE can be induced with clinical and pathological manifestations reminiscent of MS in humans. EAE is a well accepted model for MS.

Experimental autoimmune thyroiditis (EAT) can be induced in genetically susceptible strains of mice by immunizing them with mouse thyroid tissue extract or thyroglobulin (Tg) in CFA. EAT is expressed as chronic inflammation of the thyroid gland. EAT appears to be a model for autoimmune thyroiditis-Hashimoto's thyroiditis.

Collagen Type II induced arthritis (CIA) is induced by immunizing genetically susceptible strains of mice with native Type II collagen in Freund's adjuvant. CIA is manifested clinically by swelling of joints and pathologically by massive cellular infiltration into synovial tissue. CIA is one of the recently introduced models for polyarthritis.

Example 1

Reverse of clinical EAE by macrophages or B-cells pulsed with mouse spinal cord homogenate (MSCH) or with purified MBP or with encephalitogenic peptide

Cells to be pulsed with the organ homogenate can be macrophages, B cells, dendritic cells, endothelial cells, astrocytes, epithelial cells or other cells that have been adapted or induced to express MHC molecules and are capable of antigen processing and presentation to T lymphocytes. The cells have to be autologous or syngeneic in their MHC, especially in their class II MHC, with the patient. Here, for example, macrophages, astrocytes and LPS-stimulated B-cells are used for the treatment of EAE in SJL/J mice: Macrophages obtained from the peritoneal exudate of SJL/J mice injected with thioglycolate and gamma interferon to induce higher expression of MHC molecules. The macrophages were washed in PBS, and

incubated (1 x $10^7$/ml) with gamma-interferon (200 $\mu$/ml) for induction of class II MHC molecules, and with MSCH (0.3 mg/ml), or with purified antigens (50 ug/ml) or with peptide (10 ug/ml) for 6 h. LPS-stimulated cells were obtained from SJL/J spleen cells as mentioned above and freed of dead cells and cell debris by centrifugation over Ficoll as accepted.

Macrophages, LPS-stimulated B-cells pulsed with MSCH homogenate or with purified MBP or synthetic peptide were irradiated (3000 rads) and injected twice intravenously, or intraperitoneally into SJL/J mice induced for the development of EAE by MSCH in CFA, on days 3 and 7 after the encephalitogenic challenge. Under such conditions, only the treatment with macrophages pulsed either with MSCH or purified MBP or with the peptide was effective in reversing the clinical manifestation of EAE in SJL/J mice, and only when the pulsed macrophages were injected into the peritoneal cavity. An example for the results of treatment with macrophages or B cells pulsed with MSCH or MBP are presented in Table 1. The treatment with MSCH-pulsed astrocytes gave similar results to the treatment with MSCH-pulsed M$\phi$. The LPS-stimulated B cells pulsed with MSCH or MBP were not as effective under such conditions (Table 1). Presumably, the LPS-stimulated B-cells were capable of migrating from the peritoneal cavity into the circulation.

## Example 2

### Glutaraldehyde (GA) treated MSCH-pulsed cells retain their function in reversal of EAE

To avoid possible migration of cells injected intraperitoneally into circulation and also to avoid injecting viable cells that were treated extracorporeally, a process which may involve biohazards, the brain-homogenate-pulsed macrophages or B cells, or cells pulsed with MBP or with the peptide, as above, were glutaraldehyde-fixed (0.05%) prior to their intraperitoneal inoculation in a similar manner to Example 1. GA fixation was carried out by incubating the irradiated pulsed cells (5 x $10^6$/ml) in PBS containing 0.05% glutaraldehyde for 30 s at room temperature and an equal volume of 0.1 M lysine was then added. Cells were washed and resuspended in PBS and injected into mice at days 3 and 7 after mice were immunized with MSCH in CFA to develop EAE. The results in Table 2 show that the glutaraldehyde fixation did not impair the cpability of the M$\phi$ pulsed with MSCH, MBP or peptide, to reverse the development of EAE. Rather, GA-treatment was found to improve their function in reversing the disease.

Furthermore, the effect of GA-treatment on B cells pulsed with MBP or MSCH was marked. Following their GA-treatment, the B cells pulsed with MSCH or with MBP, became effective in reversing EAE when inoculated intraperitoneally.

As mentioned above the specific target antigen is largely unknown for most human autoimmune diseases. The use of macrophages or B cells to pick up the relevant target antigen from the afflicted target tissue homogenate and to use them as active ingredient for effective treatment of the autoimmune disease regardless of our knowledge concerning the specific target antigen, is of major significance for the treatment of human autoimmune diseases. However as evident from Table 2, the method of treatment is as effective when the specific antigen or the specific pathogenic epitope of the antigen is used, indicating also a possibility to restrict the treatment to a highly specific level.

## Example 3

### Reversal of EAE in other mouse strain and in Lewis rats

For the treatment of human organ-specific autoimmune diseases, the possibility to use cells pulsed with the target organ tissue homogenate is of major significance. To find out if the successful treatment of EAE by macrophages or B cells pulsed with MSCH is not unique for unknown reasons to the SJL/J mice, macrophages were prepared from (SJL/J x BALB/c)F$_1$ mice and from Lewis rats, susceptible to induction of EAE. The macrophages were pulsed with MSCH of mice, rats or of guinea pigs and GA-treated in a similar manner as in Example 2 and were inoculated intraperitoneally into the respective syngeneic recipients at days 3 and 7 after the encephalitogenic challenge. The results summarized in Table 3 show that a) EAE in other mouse strains and other species of laboratory animals can be treated in the same manner, and b) that the MSCH can be xenogeneic hence, it may well be possible to use tissue homogenate from an organ of other mammals, which is equivalent to the afflicted organ in humans, for the treatment of organ- specific autoimmune disease in humans.

In a similar manner to the treatment of EAE, other organ specific autoimmune diseases can also be treated.

Exampe 4

Reversal of other autoimmune diseaes in mice

$C_3H$ and DBA/1 mice, are genetically susceptible to induction of EAT and CIA respectively. Thyroglobulin (Tg) extraction and purification from mouse thyroid glands was prepared as described (14). Collagen type II was prepared from a rat collagen secreting chondrosarcoma (15). $C_3H$ mice were immunized twice at 7-day intervals with thyroglobulin emulsified in CFA enriched with M. Tuberculosis (7 mg/ml) for the development of EAT. CBA/1 mice were immunized twice with rat collagen type II emulsified in CFA at 2-week intervals for the induction of CIA. $C_3H$ or DBA/1 macrophages were pulsed with Tg or with collagen, respectively, and treated with glutaraldehyde as above, and were injected twice intraperitoneally into the respective mice developing EAT or CIA at 10-day intervals after the last thyroidogenic or artherogenic inoculum. For evaluating the development of EAT, the thyroid glands were removed 6 weeks after the second injection of Tg/CFA and were histologically examained for cellular inflammination in thyroid tissue as a measure for thyroiditis. The development of CIA in the immunized untreated or treated mice, was inspected daily for the onset of arthritis which was assessed by a combination of caliper measurement and visual appearance of the affected joints.

The results in Table 4 summarize the effect of the treatment of mice developing EAT or CIA by macrophages pulsed with the respective thyriodogenic or artherogenic antigen. Furthermore, the results demonstrate again the possibility to treat an organ-specific autoimmune disease by macrophages pulsed with the whole tissue extract from the inflicted organ (Table 4). The efficiency of the treatment of EAT by syngeneic macrophages pulsed with purified Tg was comparable to the treatment of EAT by macrophages pulsed with the whole thyroid gland extract.

In a similar manner to the treatment of EAE by Mφ or B cells pulsed with MBP or with MSCH, there can be achieved treatment of the disease by using only membranes of the cells rather than the whole cells.

Example 5

Reversal of EAE bv membranes

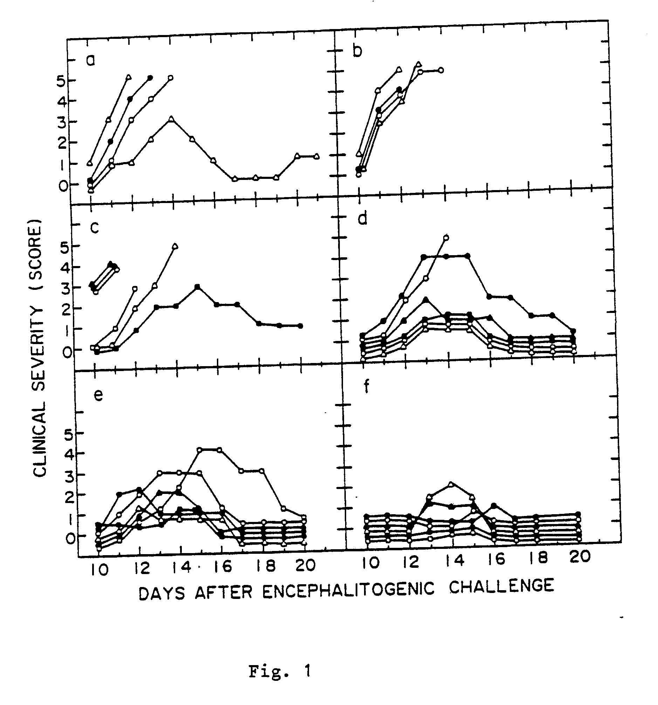

Membranes prepared from SJL/J macrophages or astrocytes that were pulsed with MBP or with MSCH, were injected into SJL/J mice immunized to develop EAE. Membranes (equivalent to 4 x $10^6$ macrophages) were inoculated intraperitoneally or intravenously on days 3 and 7 after the encephalitogenic challenge. As demonstrated in Figure 1, intraperitoneal injection of membranes from MBP-pulsed macrophages have markedly reversed the development EAE (Figure 1d), as compared to untreated mice (Fig 1a) or to control group injected intraperitoneally with membranes from unpulsed macrophages (Fig. 1b). Intravenously inoculated membranes from macrophages pulsed with MBP had no protective effect (Fig. 1c). Membranes prepared from astrocytes pulsed with MBP gave similar results.

In a similar manner to the treatment of EAE with membranes, there can be achieved more effective treatment of the disease by using modified membranes.

Example 6

Reversal of EAE by cell-surface modified membranes

Mφ, astrocytes or B cells from SJL/J mice were pulsed with MBP or MSCH and treated with glutaraldehyde as in Example 2. The cells were then subjected to a mild treatment of an oxidative agent. To oxidize cell-surface membranes the cells were incubated for 30 min. in ice-cold PBS pH 7.4 containing 1 mM sodium periodate (alternatively they were oxidized by neuraminidase and galactose oxidase by conventional methods). The cells were then extensively washed and subjected to membrane preparation after homogenization by sonication. SJL/J mice immunized to develop EAE were injected intraperitoneally or intravenously on days 3 and 7 postencephalitogenic challenge, with MBP-pulsed macrophages that had been glutaraldehyde treated and oxidized by sodium periodate. Fig. 1f shows that intraperitoneal administration of the modified MBP-pulsed macrophages resulted in the almost total abolishment of EAE development. Furthermore, the inoculation of these cells intravenously had a highly significant blocking effect on the development of EAE. As shown in Fig. 1e, none of the mice died of the disease, and out of six treated mice, only one developed severe paralysis and three developed only mild clinical symptoms.

Similarly, membranes prepared from oxidized macarophages pulsed with MSCH have also demonstrated an improved efficiency in reversing the development of EAE.

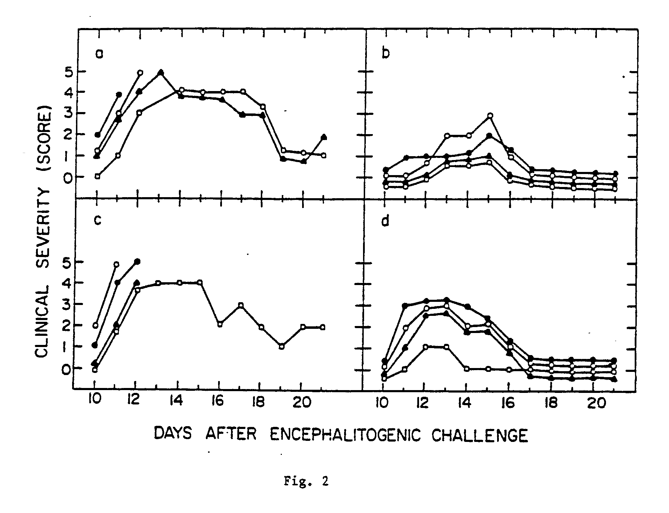

Figure 2 shows that membranes from glutarldehyde-treated and oxidized macrophages that had been pulsed with MSCH had markedly reversed the development of EAE when injected either intraperitoneally (Fig 2b) or intravenously (Fig. 2d) into SJL/J mice induced to develope EAE.

Control membranes prepared from unpulsed macrophages treated with gluraraldehyde and sodium periodate had no significant effect on the development of EAE (Fig. 2a). Membranes prepared from MSCH-pulsed macrohpages, but not oxidized by sodium periodate, when inoculated intravenously into SJL/J mice induced to develop EAE, failed to reverse EAE development (Fig. 2c).

Hence, oxidizing cell-surface membranes, facilitated the reverseal of EAE development by cells or membranes also when injected intravenously. The mechanism by which cell-surface oxidation facilitates the capability of cells or membranes to reverse EAE is not yet known. It is believed, however, that oxidized glycoproteins on the cell-surface membrane, may contribute in further stabilizing the interaction between the membranes and the pathogenic T lymphocytes interacting with the membranes via the T cell receptor.

In a similar manner to the treatment of EAE by intraperitoneal or intravenous inoculation of cells or membranes, there can be achieved treatment of the disease by oral or subcutaneous administration or by aerosol application.

## Example 7

SJL/J macrophages or LPS-stimulated B cells were pulsed with MBP or with MSCH and treated with glutaraldehyde. The cells ($5 \times 10^6$ or $10 \times 10^6$ respecitvely), or membranes prepared thereof, were introduced either orally or by subcutaneous injections into SJL/J mice on days 3 and 7 after they had been immunized to develop EAE. The results shown in Table 5 demonstrate the effect of such administration of cells or membranes on the development of EAE. Compared with 100% incidence of paralysis in control groups, only 28% of mice in the orally treated groups combined, develped clinical signs of EAE and the disease was milder. Furthermore, out of the 50 mice treated orally combined, only two mice died of the disease (4%) as compared to 83% mortality in control groups. Similarly, although the less effectively, the administration of cells or membranes subcutaneously also had a significant protective effect (50%) against EAE development, and 60% protection against mortality from EAE.

A suitable active ingredient according to the invention can be applied in aerosol form.

The above defined pharmaceutical compositions are of value in humans and veterinary medicine. In human medicine dose forms based on about 1-100 $\times 10^6$ cells are applied.

## Legend to figures

Fig. 1. Reversal of EAE by membrane-associated MBP. SJL/J mice induced for the development of EAE were either not treated (a) or treated, on days 3 and 7 after the encephalitogenic challange, with membranes prepared from unpulsed macrophages inoculated i.p.(b), or prepared from MBP-pulsed macrophages and inocluated i.v. (c) or i.p. (d), or with the whole MBP-pulsed macrophages that were GA-treated and oxidized by sodium periodate and inoculated i.v.(e) or i.p. (f). $2 \times 10^6$ Cells or membranes equivalent to $4 \times 10^6$ cells were inoculated into each recipient. Each symbol in each panel represents an individual mouse of the treatment group. Mice were observed daily for the development of clinical signs of EAE and each mouse was scored daily as follows; 1, partial loss of tail tone; 2, tail paralysis and weakness of one hind limb; 3, paralysis of both hind limbs; 4, paralysis of hind and fore limbs; 5, premorbid state.

Fig. 2. Treatment of EAE with modified membranes. Membranes, equivalent to $4 \times 10^6$ cells were

injected into SJL/J mice developing EAE, on days 3 and 7 after encephalitogenic challenge.

(a) i.p. injection with membranes from unpulsed macrophages treated for cross-linking and cell-surface oxidation by sodium periodate. (b) i.p. or (d) i.v. inoculation of membranes from MSCH-pulsed macrophages treated for cross- linking and oxidation, and (c) were injected with membranes from MSCH-pulsed macrophages that were not oxidized. Clinical score is as in legend to Fig.1.

References

1. Paterson, P.Y. and Dorbish, P., Science 165:191 (1969).

2. Brostoff, S.W. and Mason D.W., J.Immunol. 133:1938 (1984).

3. Waldor, M.K., Sriram, S., McDevitt, H.O., Steinman, L., Proc.Natl.Acad.Sci. 80:2713 (1983).

4. Levine, S., Honing, E.M., Kies, M., Science 1612:1155 (1968).

5. Teitelbaum D., Webb, C., Bree, M., Meshorer, A., Arnon, R., Sela, M., Clin.Immunol.Immunopathol. 3:256 (1974).

6. Ben-Nun, A., Wekerle, H., Cohen, I.R., Nature 292:60, (1981).

7. Acha-Orbea, H., Mitchell, D.J., Timmermann, L., Wraith, D.C., Tauch, G.S., Waldor, M.K., Zamvil, S.S., McDevitt, H.O., Steinmenn, L., Cell 54:263 (1988).

8. Urbain, J.L., Kumor, V., Kono, D.H., Gomez, G., Horvath, S., Clayton, J., Ando, P.G., Sercarz, E.E., Hood, L., Cell, 54:577 (1988).

9. Vandenbark, A.A., Hashim, G., Offner, H., Nature 341:541, (1989).

10. Howsell, M.D>, Winster, S.T., Olee, T., Powell, H.C., Carlo, D.J., Brostoff, S.W., Science 246:668 (1989).

11. Urban, J.L., Horvath, S.J., Hood, L., Cell 59:257, (1989).

12. Wairth, D.C., Smilek, D.E., Mitchell, D.J., Steinman, L., mcDevitt, M.0., Cell 59:247 (1989).

13. Thom, P., Powell, A.J., Lloyd, C.W., Rees, D.A., Biochem.J. 168:187 (1977).

14. Rose, N.R., Twarog, F.J., Crowle, J.A. J.Immunol. 106:698, (1971).

15. Stuart, J.M., Cremer, M.A., Townes, A.S., Kang, A.H., Arthritis Rheum. 22:1344 (1979).

TABLE 1. Reversal of EAE development by MBP-pulsed macrophages

| | Incidence of EAE in treated mice | | | | | |
|---|---|---|---|---|---|---|
| | I.v. | | | I.p. | | |
| Treatment | Paral-ysis | Mean score | Mortality | Paral-ysis | Mean score | Mortality |
| PBS | 19/20 | 4.2 | 14/20 | 18/20 | 4.2 | 15/20 |
| MBP | 5/5 | 3.8 | 4/5 | 5/5 | 3.9 | 5/5 |
| MSCH | 7/7 | 4.4 | 7/7 | 7/7 | 3.8 | 6/7 |
| Unpulsed MΦ | n.t.[a] | - | - | 7/7 | 3.9 | 5/7 |
| MBP-pulsed MΦ: | | | | | | |
| $2 \times 10^6$ | 6/6 | 4.4 | 6/6 | 5/10 | 2.2 | 2/10 |
| $0.5 \times 10^6$ | 6/6 | 4.2 | 5/6 | 4/6 | 2.2 | 1/6 |
| | | | | | | <0.0001[b] (60%)[c] |
| MSCH-pulsed MΦ: | | | | | | |
| $2 \times 10^6$ | 7/7 | 3.9 | 6/7 | 5/7 | 1.8 | 3/7 |
| $0.5 \times 10^6$ | 7/7 | 4.2 | 5/7 | 4/7 | 1.7 | 2/7 |
| | | | | | | <0.008 (44%) |
| OVA-pulsed MΦ | n.t. | - | - | 6/6 | 4.3 | 5/6 |
| PPD-pulsed MΦ | n.t. | - | - | 7/7 | 2.9 | 5/7 |
| Unpulsed B cells | 6/8 | 4.0 | 6/8 | 8/8 | 4.0 | 6/8 |
| MBP-pulsed B cells: | | | | | | |
| $5 \times 10^6$ | 8/8 | 4.4 | 8/8 | 7/8 | 3.6 | 6/8 |
| $10^6$ | 7/8 | 4.2 | 7/8 | 7/8 | 2.9 | 5/8 |
| | | | | | | >0.05 (11%) |

EP 0 385 373 A2

## Table 1, continued

SJL/J mice were treated on day 3 and 7 after the encephalitogenic challenge by injecting them i.p. or i.v. with syngeneic macrophages ($2x10^6$ cells or as indicated) or with B cells ($5x10^6$ or as indicated) that were unpulsed or pulsed with MBP or MSCH. Mean score is the mean of the maximal score of clinical signs of each mouse in the treatment group. The score of clinical signs is as described in the legend to Figure 1.

Footnotes:

[a] - n.t.; not tested

b - p values calculated by the $\chi^2$ test by comparison to control groups combined (31/39).

c - Net protection; % survival in test minus % survival in controls combined.

EP 0 385 373 A2

TABLE 2. Glutaraldehyde treated MBP-pulsed cells retain their function in reversal of EAE

| | Incidence of EAE in treated mice | | | | | |
| | I.v. | | | I.p. | | |
| Treatment | Paralysis | Mean score | Mortality | Paralysis | Mean score | Mortality |
|---|---|---|---|---|---|---|
| None | 10/10 | 4.0 | 8/10 | - | - | - |
| Unpulsed MΦ, GA | 6/6 | 4.2 | 5/6 | 6/6 | 3.9 | 5/6 |
| MBP-pulsed MΦ, GA | 6/6 | 4.0 | 6/6 | 3/6 | 1.2 | 0/6 $<10^{-6}$[b] (79%)[c] |
| MBP peptide-pulsed MΦ, GA[d] | n.t.[a] | | | 3/6 | 1.5 | 1/6 $<10^{-5}$ (63%) |
| MSCH-pulsed MΦ, GA | 6/6 | 4.5 | 5/6 | 3/6 | 2.0 | 1/6 $<10^{-5}$ (63%) |
| Unpulsed B cells, GA | 7/7 | 3.7 | 5/7 | 7/7 | 3.6 | 6/7 |
| MBP-pulsed B cells, GA | 7/7 | 3.9 | 6/7 | 5/7 | 1.8 | 2/7 $<10^{-5}$ (51%) |
| MSCH-pulsed B cells, GA | n.t. | - | - | 7/7 | 2.1 | 3/7 $<10^{-4}$ (37%) |
| MBP-pulsed allogeneic[e] | | | | | | |
| B cells, GA | n.t. | - | - | 5/5 | 3.8 | 4/5 |
| MΦ, GA | n.t. | - | - | 5/5 | 3.9 | 4/5 |

Table 2, continued

## LEGEND

SJL/J mice were treated with MBP- or MSCII-pulsed cells as in Table 1, except cells were treated with glutaraldehyde (GA) prior to inoculation.

## FOOTNOTES
a, b and c as in footnotes to Table 1.

[d]Encephalitogenic synthetic peptide encompassing 89-101 aa residues of MBP...

[e]Cells were from B10.BR mice.

EP 0 385 373 A2

Table 3.

| Reversal of EAE in Lewis rats and (SJL/J x BALB/c)F1 mice | | | | | | |
|---|---|---|---|---|---|---|
| | Incidence of EAE | | | | | |
| | Lewis rats | | | (SJL/JxBALB/c)F1 mice | | |
| Treatment | Paralysis | Mean Score | Mortality | Paralysis | Mean Score | Mortality |
| PBS | 7/8 | 3.5 | 1/8 | 10/10 | 3.9 | 6/10 |
| OVA | 8/8 | 3.2 | 2/8 | 9/10 | 3.4 | 5/10 |
| MBP | 8/8 | 3.4 | 1/8 | 10/10 | 3.5 | 5/10 |
| Unpulsed M$\phi$, GA | 6/7 | 3.2 | 2/7 | 8/8 | 3.6 | 5/8 |
| Rat MBP-pulsed M$\phi$, GA | 2/7 | 1.6 | 0/7 | 4/10 | 1.8 | 0/10 |
| Rat MSCH-pulsed M$\phi$, GA | 2/7 | 1.8 | 0/7 | 4/10 | 1.5 | 0/10 |
| Mouse MSCH-pulsed M$\phi$, GA | 3/7 | 1.7 | 0/7 | 5/10 | 1.7 | 0/10 |
| Guinea pig MSCH-pulsed M$\phi$, GA | 2/7 | 1.9 | 0/7 | 6/10 | 1.5 | 0/10 |
| | $<10^{-4}$(70%) | | | | | $<10^{-5}$(45%) |

Macrophages (M∅) from Lewis rats or (SJL/J x BALB/c)F1 mice were unpulsed or pulsed with MBP or MSCH and treated with glutaraldehyde (GA). On days 3 and 7 after encephalitogenic challenge, the cells ($2 \times 10^5$) were injected intrape itoneally into the respective syngeneic recipients. Control OVA and MBP (50$\mu$g) were injected in soluble form intraperitoneally.

Table 4:

| Treatment of experimental autoimmune thyroiditis (EAT) and collagen (type II) induced arthritis (CIA) | | | | |
|---|---|---|---|---|
| Treatment with GA-treated Cells | EAT in C3H mice | | CIA in DBA/1 mice | |
| | Incidence | % protection (net) | Incidence | % protection (net) |
| Untreated | 12/15 | | 7/12 | |
| Unpulsed C3H M$\phi$ | 13/15 | | -- | |
| Unpulsed DBA/1 M$\phi$ | -- | | 7/12 | |
| Tg-pulsed C3H M$\phi$ | 2/15 | 87 (70) | -- | |
| Collagen-pulsed CBA/1, M$\phi$ | -- | | 0/12 | 100 (58) |
| C3H M$\phi$ pulsed with thyroid gland extract | 1/15 | 94 (77) | NT | |

M∅ from C3H mice unpulsed or pulsed with thyroglobulin (Tg) or with thyroid gland extract were injected twice intraperitoneally into C3H mice at 7-day intervals after last injection of Tg/CFA for induction of thyroiditis. M∅ from DBA/1 pul ed or unpulsed with collagen were injected intraperitoneally at 10-day intervals after last injection of collagen in CFA for the induction of CIA.

EP 0 385 373 A2

Table 5:

| Treatment of EAE by oral or subcutane administration | | | | |
|---|---|---|---|---|
| | Incidence of EAE | | | |
| | Oral | | Subcutane | |
| Treatment | Paralysis | Mortality | Paralysis | Mortality |
| Untreated | 10/10 | 8/10 | - | - |
| PBS | 10/10 | 9/10 | 10/10 | 8/10 |
| MBP | 9/10 | 8/10 | 9/10 | 8/10 |
| Unpulsed | 9/10 | 8/10 | 10/10 | 9/10 |
| MBP-pulsed B cells, GA | 3/10 | 1/10 | 5/10 | 3/10 |
| MSCH-pulsed Mφ, GA | 2/10 | 0/10 | 5/10 | 4/10 |
| MBP-pulsed B cells, GA | 3/10 | 0/10 | NT | |
| MSCH-pulsed B cells, GA | 3/10 | 0/10 | NT | |
| Membranes from MSCH-pulsed Mφ, GA | 3/10 | 1/10 | 5/10 | 4/10 |

MBP- or MSCH-pulsed MØ ($5 \times 10^6$) or B cells ($10 \times 10^6$) treated with glutaraldehyde (GA) or membranes (equivalent to $5 \times 10^6$ cells) were administered orally or subcutnaeously on the back on days 3 and 7 after SJ /J mice were immunized to develop EAE. Fifty μg MBP was administered in a soluble form.

## Claims

1. A pharmaceutical composition for the treatment of, and for the prevention of autoimmune disease in humans which comprises as active ingredient an effective quantity of MHC molecules expressing cells of the patient, or of membranes or membrane fragments of such cells or of cells which are compatible with such cells, which cells or membranes present at their surface, antigens specific for such autoimmune disease, said antigens being derived from a tissue homogenate, a total or partial tissue extract, or purified protein, or antigen, or fragments thereof specific for such autoimmune disease, which cells or membranes or membrane fragments have optionally been subjected to oxidation or cross-linking or both.

2. A composition according to claim 1, where the MHC presenting cells are selected from B lymphocytes, macrophages, endothelial cells, dendritic cells, astrocytes and other cells expressing MHC molecules or adapted to express MHC molecules, to process and to present at their surface, antigens for T lymphocytes.

3. A composition according to claim 1 or 2, intended for the treatment of an autoimmune disease which is organ-specific, and where the antigen is derived from such organ.

4. A composition according to any of claims 1 to 3, where the cells used have been subjected to an oxidataive treatment.

5. A composition according to any of claims 1 to 4, where the cells have been cross-linked by a suitable agent.

6. A composition according to any of claims 1 to 5, where instead of whole cells, membranes or membrane fragments of such cells are used.

7. Pharmaceutical compositions for the prevention and for the treatment of autoimmunne diseases, substantially as hereinbefore described and with references to any of the Examples and Tables or Graphs.

8. A process for the production of MHC molecules expressing cells or membranes, capable of presenting antigens to lymphocytes involved in the disease, for use as active ingredient in a composition claimed in any of claims 1 to 7, which comprises incubating a tissue homogenate or extract or a protein fraction, total or partial, from the afflicted organ of a patient, or from an equivalent organ of another mammal or a purified protein or antigen, or a fragment thereof, with said cells or cells adapted to present at their surface antigens for T-lyphocytes which cells or membranes or membrane fractions have optionally been subjected to oxidation or cross-linking or both.

9. A process according to claim 8, where the cells are selected from B lymphocytes, macrophage, endothelial cells, dendritic cells, astrocytes and other cells adapted to express MHC molecules and to

15

present at their surface, atigens for T lymphocytes.

10. A process according to claim 8 or 9, which comprises subjecting such cells to a further step of oxidative treatment.

11. A process according to any of claims 8 to 10, which comprises subjecting such cells to the additional step of cross-linking.

12. A process according to claim 11, where the cross-linking is effected with a suitable agent.

13. A process according to any of claims 8 to 12, where instead of cells only the cell membrane or fragments is used.

14. A process according to any of claims 8 to 13, where the organ extract is produced by homogenizing the tissue and discarding undesired tissue components.

15. A pharmaceutical composition according to any of claims 1 to 8, in injectable dosage form.

16. A parmaceutical composition according to any of claims 1 to 8, in an aerosol dosage form.

17. A pharmaceutical composition according to any of claims 1 to 8, in an oral dosage form.

18. Process for the production of the cells defined in claim 1, substantially as hereinbefore described and with reference to any of the Examples or Figures.

Claims For The following Contracting State: ES

1. A process for preparing a pharmaceutical composition for the treatment of, and for the prevention of autoimmune disease in humans which comprises mixing as active ingredient an effective quantity of MHC molecules expressing cells of the patient, or of membranes or membrane fragments of such cells or of cells which are compatible with such cells, which cells or membranes present at their surface, antigens specific for such autoimmune disease, said antigens being derived from a tissue homogenate, a total or partial tissue extract, or purified protein, or antigen, or fragments thereof specific for such autoimmune disease, which cells or membranes or membrane fragments have optionally been subjected to oxidation or cross-linking or both and a pharmaceutically acceptable carrier and/or diluent.

2. A process according to claim 1, where the MHC presenting cells are selected from B lymphocytes, macrophages, endothelial cells, dendritic cells, astrocytes and other cells expressing MHC molecules or adapted to express MHC molecules, to process and to present at their surface, antigens for T lymphocytes.

3. A process according to claim 1 or 2, intended for the treatment of an autoimmune disease which is organ-specific, and where the antigen is derived from such organ.

4. A process . according to any of claims 1 to 3, where the cells used have been subjected to an oxidataive treatment.

5. A process according to any of claims 1 to 4, where the cells have been cross-linked by a suitable agent.

6. A process according to any of claims 1 to 5, where instead of whole cells, membranes or membrane fragments of such cells are used.

7. A process for the production of MHC molecules expressing cells or membranes, capable of presenting antigens to lymphocytes involved in the disease, for use as active ingredient in a composition claimed in any of claims 1 to 7, which comprises incubating a tissue homogenate or extract or a protein fraction, total or partial, from the afflicted organ of a patient, or from an equivalent organ of another mammal or a purified protein or antigen, or a fragment thereof, with said cells or cells adapted to present at their surface antigens for T-lyphocytes which cells or membranes or membrane fractions have optionally been subjected to oxidation or cross-linking or both.

8. A process according to claim 7, where the cells are selected from B lymphocytes, macrophage, endothelial cells, dendritic cells, astrocytes and other cells adapted to express MHC molecules and to present at their surface, atigens for T lymphocytes.

9. A process according to claim 7 or 8, which comprises subjecting such cells to a further step of oxidative treatment.

10. A process according to any of claims 7 to 9, which comprises subjecting such cells to the additional step of cross-linking.

11. A process according to claim 10, where the cross-linking is effected with a suitable agent.

12. A process according to any of claims 7 to 11, where instead of cells only the cell membrane or fragments is used.

13. A process according to any of claims 7 to 12, where the organ extract is produced by homogenizing the tissue and discarding undesired tissue components.

14. A process according to any of claims 1 to 7, characterized in that the product is obtained in injectable dosage form.

15. A process according to any of claims 1 to 7, characterized in that the product is obtained in an aerosol dosage form.

16. A process according to any of claims 1 to 7, characterized in that the product is obtained in an oral dosage form.

17. Process for the production of the cells defined in claim 1, substantially as hereinbefore described and with reference to any of the Examples or Figures.

Fig. 1

Fig. 2